(19) Europäisches Patentamt
European Patent Office
Office européen des brevets

(11) Veröffentlichungsnummer: **0 496 238 A1**

(12) # EUROPÄISCHE PATENTANMELDUNG

(21) Anmeldenummer: **92100415.6**

(22) Anmeldetag: **13.01.92**

(51) Int. Cl.5: **C07D 491/08**, C07D 495/08, C07D 311/22, C07D 335/06, A61K 31/55, //(C07D491/08, 311:00,209:00),(C07D495/08, 335:00,209:00)

(30) Priorität: **25.01.91 DE 4102103**

(43) Veröffentlichungstag der Anmeldung: **29.07.92 Patentblatt 92/31**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Kabbe, Hans-Joachim, Dr.**
**Walter-Flex-Strasse 16**
**W-5090 Leverkusen(DE)**
Erfinder: **Heitzer, Helmut, Dr.**
**Höhenstrasse 84**
**W-5090 Leverkusen 3(DE)**
Erfinder: **Knorr, Andreas, Dr.**
**Trillser Graben 10**
**W-4006 Erkrath 2(DE)**
Erfinder: **Hirth-Dietrich, Claudia, Dr.**
**Claudiusweg 9**
**W-5600 Wuppertal(DE)**

(54) **Substituierte Benzoxazepine und Benzthiazepine, Verfahren zu ihrer Herstellung und ihre Verwendung in Arzneimitteln.**

(57) Die vorliegende Erfindung betrifft neue substituierte Benzoxazepine und Benzthiazepine der allgemeinen Formel I

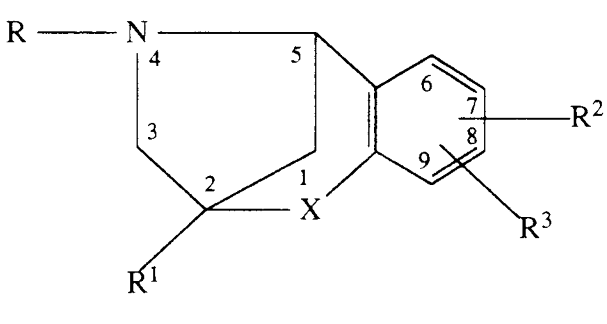

(I)

in welcher X, $R^1$, $R^2$ und $R^3$ die in der Beschreibung angegebene Bedeutung haben. Verfahren zu ihrer Herstellung und ihre Verwendung in Arzneimitteln mit gefäß-und muskelrelaxierenden Eigenschaften, insbesondere als Kreislaufmittel.

EP 0 496 238 A1

Die vorliegende Erfindung betrifft neue substituierte Benzoxazepine und Benzthiazepine, Verfahren zu ihrer Herstellung und ihre Verwendung in Arzneimitteln mit gefäß- und muskelrelaxierenden Eigenschaften, insbesondere als Kreislaufmittel.

Es sind bereits Wirkstoffe mit gefäß- und muskelrelaxierenden Wirkungen bekannt, die teilweise auch als Kreislaufmittel eingesetzt werden (vgl. EP 0 388 528). Die Verbindungen aus dem Stand der Technik unterscheiden sich jedoch von den erfindungsgemäßen Verbindungen eindeutig sowohl in ihrer chemischen Struktur als auch in ihrem Wirkungsprofil.

Die Erfindung betrifft neue 2,5-Methano-benzoxazepine und -benzthiazepine der allgemeinen Formel (I)

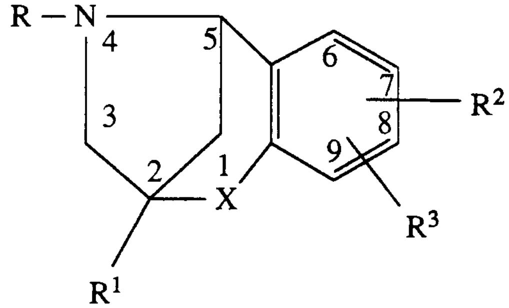

,

in welcher

| | |
|---|---|
| X | für Sauerstoff oder Schwefel steht, |
| R | für Wasserstoff oder eine der Gruppen $COR^4$, $CO\text{-}NHR^4$, $CS\text{-}NHR^4$, $CO\text{-}OR^4$ oder $SO_2R^4$ steht, wobei |
| $R^4$ | jeweils für Wasserstoff, geradkettiges, verzweigtes oder cyclisches Alkyl mit bis zu 7 C-Atomen, für Alkenyl mit 2 bis 4 C-Atomen, für Phenalkyl mit 7 bis 10 C-Atomen, für Phenyl oder für einen 5- oder 6-gliedrigen Heteroarylrest steht, der ein oder zwei gleiche oder verschiedene Heteroatome aus der Gruppe Sauerstoff, Schwefel oder Stickstoff enthält und wobei die genannten Alkylreste gegebenenfalls durch Halogen substituiert sind und die Phenylreste gegebenenfalls ein- oder zweifach durch Alkyl oder Alkoxy mit jeweils 1 bis 4 C-Atomen oder durch Halogen oder Nitro substituiert sind, |
| $R^1$ | für Wasserstoff oder Alkyl mit 1 bis 4 C-Atomen steht, |
| $R^2$ and $R^3$ | gleich oder verschieden sind und jeweils für ein oder zwei Substituenten aus der Gruppe geradkettiges, verzweigtes oder cyclisches Alkyl mit 1 bis 7 C-Atomen, das gegebenenfalls durch Halogen substituiert ist, Alkoxy mit 1 bis 6 C-Atomen, Halogen, Nitro, Carboxy, Hydroxy, Carbonamid, Alkoxycarbonyl mit bis zu 7 C-Atomen, Alkylsulfonyl mit 1 bis 6 C-Atomen, Phenylsulfonyl oder einen 5- oder 6-gliedrigen Heteroarylrest, der ein oder zwei Heteroatome aus der Gruppe Stickstoff, Sauerstoff oder Schwefel enthält, für Phenyl, welches gegebenenfalls ein- oder zweifach substituiert ist durch Alkyl oder Alkoxy mit 1 bis 4 C-Atomen, Halogen oder Nitro, oder für $COR^4$ stehen, |

oder

$R^2$ gemeinsam mit $R^3$ einen 3- oder 4-gliedrigen anellierten Ring bildet,

sowohl in Form von Isomerengemischen als auch in isomerenreiner Form.

Die erfindungsgemäßen Verbindungen erniedrigen den Kationengehalt in Muskel-und Gefäßzellen. Insbesondere werden die Calcium- und Kaliumionen positiv beeinflußt. Vorzugsweise wirken die erfindungsgemäßen Verbindungen relaxierend auf Blutgefäße und Bronchien und können somit zur Behandlung von Kreislauferkrankungen, insbesondere Hochdruck und Asthma verwendet werden.

Von besonderer Bedeutung sind Verbindungen der allgemeinen Formel (I),

in welcher

| | |
|---|---|
| X | für Sauerstoff oder Schwefel steht, |
| R | für Wasserstoff, $COR^4$, $CONHR^4$, $CSNHR^4$, $COOR^4$ oder $SO_2R^4$ steht, wobei |
| $R^4$ | für Alkyl oder Alkoxy mit bis zu 4 C-Atomen steht, wobei Alkyl gegebenenfalls durch Fluor oder Chlor substituiert ist, oder für Phenyl steht, das gegebenenfalls durch Alkyl oder Alkoxy mit 1 bis 4 C-Atomen, Fluor, Chlor oder Nitro ein- oder zweifach substituiert ist, oder für Heteroaryl aus der Gruppe Thiophen, Pyridin oder Furan, die gegebenenfalls |

substituiert sind durch Fluor, Chlor, Methyl, Ethyl oder Methoxy,

$R^1$ für Wasserstoff oder Alkyl mit 1 bis 4 C-Atomen steht,

$R^2$ und $R^3$ gleich oder verschieden sind und jeweils für ein oder zwei Substituenten aus der Gruppe Wasserstoff, Halogen, Nitro, Carboxy, Cyano, Hydroxy, Carbonamid, Phenyl, Phenylsulfonyl, Alkyl mit 1 bis 4 C-Atomen, Alkoxy mit 1 bis 4 C-Atomen, Alkoxycarbonyl mit bis zu 5 C-Atomen, Alkylsulfonyl mit 1 bis 4 C-Atomen, Thiophen, Pyridin oder Furan stehen, wobei die genannten Alkyl-, Aryl- und Heteroarylreste gegebenenfalls substituiert sind durch Fluor, Chlor, Nitro, Alkyl mit 1 bis 4 C-Atomen oder Alkoxy mit 1 bis 4 C-Atomen, oder

$R^2$ und $R^3$ gemeinsam einen 3- oder 4-gliedrigen anellierten Ring bilden.

Besonders bevorzugt sind Verbindungen der allgemeinen Formel (I), in welcher

X für Sauerstoff steht,

R für Wasserstoff, $CO-R^4$, $CO-NH-R^4$, $COOR^4$ oder $SO_2-R^4$ steht, wobei

$R^4$ für Alkyl oder Alkoxy mit 1 bis 4 C-Atomen steht, wobei Alkyl durch Fluor substituiert sein kann,

$R^1$ für Alkyl mit 1 bis 4 C-Atomen steht,

$R^2$ und $R^3$ gleich oder verschieden sind und jeweils für 1 bis 2 Substituenten aus der Gruppe Wasserstoff, Halogen, Nitro, Cyano, Alkyl mit bis zu 4 C-Atomen, Alkylsulfonyl mit bis zu 4 C-Atomen stehen, wobei die genannten Alkylreste durch Fluorsubstituiert sein können.

Die erfindungsgemäßen Verbindungen der allgemeinen Formel (I) können hergestellt werden, indem man Verbindungen der allgemeinen Formel (II)

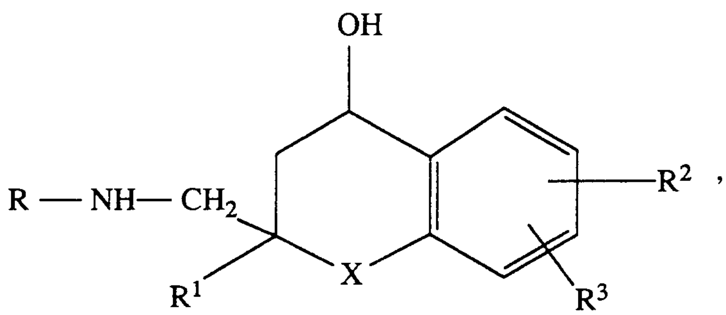

in welcher

X, R, $R^1$, $R^2$ und $R^3$ die oben angegebene Bedeutung haben,

bei Temperaturen zwischen 60 und 200 °C, vorzugsweise zwischen 70 und 140 °C, in inerten organischen Lösungsmitteln in Gegenwart von Säuren kondensiert, wobei gegebenenfalls das entstehende Wasser azeotrop abdestilliert wird.

Als Säuren verwendet man vorzugsweise hochsiedende wasserfreie Säuren wie Schwefelsäure, Alkyl-, Aralkyl- oder Arylsulfonsäuren mit bis zu 10 C-Atomen oder Phosphorsäure.

Als organische Lösungsmittel verwendet man vorzugsweise solche, die gegenüber den Reaktionspartnern inert sind, die bei der Reaktionstemperatur sieden und das Wasser somit azeotrop entfernen können, wie z.B. Kohlenwasserstoffe, wie Cyclohexan, Petrolether, Benzol, Toluol oder Xylol, Halogenkohlenwasserstoffe, wie Chlorbenzol oder Dichlorbenzol, Ester wie Essigsäurebutylester oder Gemische der genannten Lösungsmittel.

Die Herstellung der Ausgangsverbindungen der Formel (II) erfolgt nach üblichen Methoden, z.B. durch Cyclisierung von o-Hydroxyacetophenonderivaten der allgemeinen Formel (III)

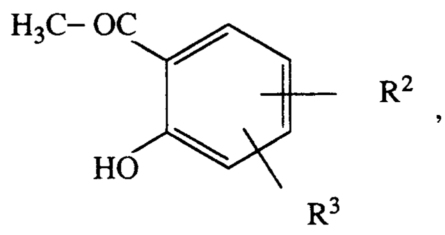

mit Ketonen der allgemeinen Formel (IV)

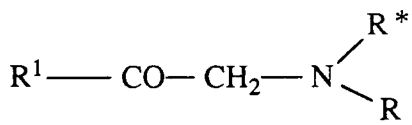

in der

R* für Wasserstoff oder einen abspaltbaren Acylrest, wie unter R definiert, steht,

zu Benzopyranonen der Formel (V)

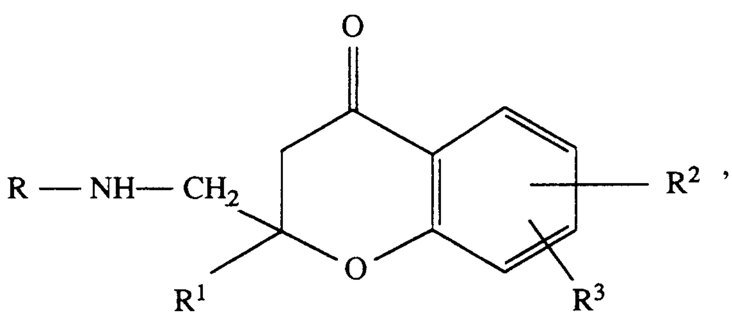

wobei

R,$R^1$,$R^2$ und $R^3$ die oben angegebene Bedeutung haben,

nach an sich bekannten Methoden (z.B. H.J. Kabbe et al., Ang. Ch. 94, 254 (1982)) und durch nachfolgende Reduktion der Verbindungen (V) nach üblichen Methoden, z.B. mit komplexen Hydriden, wie z.B. Natriumborhydrid, in inerten Lösungsmitteln, wie Alkoholen, oder durch katalytische Hydrierung in Gegenwart von Schwermetallkatalysatoren.

Die erfindungsgemäßen Verbindungen zeigen ein nicht vorhersehbares wertvolles pharmakologisches Wirkungsspektrum. Sie können deshalb zur Herstellung von Arzneimitteln zur Behandlung von Kreislauferkrankungen, insbesondere Hypertonie, oder zur Behandlung von Erkrankungen des Salzhaushaltes, von Spasmen oder zur Behandlung von Asthma eingesetzt werden.

Ihre interessanten Wirkungen lassen sich an folgendem pharmakologischen Test zeigen:

Die erfindungsgemäßen Verbindungen haben eine starke und anhaltende antihypertensive Wirkung bei Ratten mit spontaner Hypertonie.

Diese Wirkung wurde an wachen Tieren festgestellt, deren Blutdruck mit der indirekten Schwanzmikrophontechnik vor sowie 1, 2, 4, 6 und 24 Stunden nach oraler Gabe gemessen wurde.

So wurde bereits nach Dosen um 5 mg/kg eine starke und über mehrere Stunden anhaltende Blutdrucksenkung beobachtet (Beipsiel 5).

Die neuen Wirkstoffe können in bekannter Weise in übliche Formulierungen überführt werden wie Tabletten, Dragees, Pillen, Granulate, Sirupe, Aerosole, Emulsionen, Suspensionen und Lösungen. Hierbei werden inerte, nicht-toxische, pharmazeutisch geeignete Trägerstoffe oder Hilfsstoffe eingesetzt.

Ausführungsbeispiele

Beispiel I

20,2 g (0,2 m) Formylaminoaceton [A. Treibs u.a., Chem. Ber. 84 96 (1951)] und 27,2 g (0,2 m) o-Hydroxyacetophenon werden in 100 ml Toluol gelöst und mit 10 ml Pyrrolidin versetzt. Man verrührt den Ansatz 20 Stunden bei 25 ° C, dann 2 Stunden bei 115 ° C. Nach dem Abkühlen schüttelt man nacheinander mit 2n HCl, 2n NaOH und Wasser aus, engt die organische Phase ein und isoliert durch Chromatographie das gebildete 2-Methyl-2-formylaminomethylchromanon-(4). Schmp.: 96-8 ° C.

Beispiel II

142 g (0,87 m) Diacetylamino-aceton und 120 g (0,88 m) o-Hydroxyacetophenon werden in 400 ml Toluol gelöst und nach Zugabe von 185 ml Pyrrolidin 4 Tage bei 25 ° C verrührt. Nach der gleichen Aufarbeitung wie in Beispiel 1 erhält man eine Toluol-Lösung, die eingeengt wird. Nach Zugabe von Ether fallen 67,8 g 2-Methyl-2-acetylaminomethyl-chromanon-(4) aus (Schmelzpunkt: 98-100 ° C). Die vereinigten wäßrigen Extrakte werden mit n-Butanol ausgeschüttelt; die organische Phase liefert nach dem Einengen und einer chromatographischen Reinigung weitere 69 g des gleichen Chromanons.
$^1$H-NMR($CDCl_3$): 1,38 ppm (s,$CH_3$); 2,05 (s,$CH_3CO$); 2,75 (q,$CH_2$,$\delta_{AB}$ = 16,5 Hz); 3,58 (d/q, $CH_2$-NH, $\delta_{AB}$ = 14,0 Hz, $\delta_{ANH}$ = 5,5 Hz, $\delta_{BNH}$ = 7,0 Hz); 6,58 (d/d;NH); 6,9-7,9(4 aromat. H).

Beispiel III

60,9 g des in Beispiel 2 erhaltenen Chromanons werden in 250 ml Methanol gelöst. Man gibt bei unter 35 ° C 8 g Natriumborhydrid zu, rührt 24 Stunden, versetzt erneut portionsweise mit $NaBH_4$ (8 g) und rührt weitere 24 Stunden nach. Die Lösung wird eingeengt. Man versetzt den Rückstand mit Wasser und extrahiert das gebildete 2-Methyl-2-acetylaminomethyl-chromanol-(4) mit Ether und Butanol. Einengen dieser Extrakte liefert 55 g Endprodukt vom Schmp. 168-70 ° C.

Beispiel IV

20 g des im Beispiel II erhaltenen Chromanons werden mit 150 ml 2n HCl 5 Stunden zum Sieden erhitzt. Anschließend wird die Lösung mit 2n NaOH auf einen pH-Wert von über 12 gestellt und mit Chlorbenzol extrahiert. Nach dem Einengen verblieben 16,4 g eines Öls, das in 100 ml Toluol gelöst wird. Man gibt 8 g des gemischten Anhydrids aus Essigsäure und Ameisensäure hinzu, worauf die Temperatur auf 45 ° C steigt. Es wird 8 Stunden bei 60 ° C nacherwärmt und eingeengt. Aus dem Rückstand erhält man 13,5 g 2-Methyl-2-formylaminomethylchromanon-(4), das mit dem in Beispiel I gewonnen Produkt identisch ist.

Gemäß den im Vorangehenden beschriebenen Versuchen werden analog erhalten:

Beispiel V

2-Methyl-2-acetylaminomethyl-6-chlorbenzopyranon-(4):
Schmp. 111-113 ° C.

Beispiel VI

2-Methyl-2-acetylaminomethyl-7-methoxybenzopyranon-(4):
Schmp. 145-147 ° C.

Beispiel VII

2-Methyl-2-acetylaminomethyl-6-trifluormethoxybenzopyranon-(4), ölig.

Beispiel VIII

2-Methyl-2-benzoylaminomethylchromanon-(4):
Schmp. 115-117 ° C.

Beispiel IX

2-Methyl-2-acetylaminomethyl-6-chlorchromanol-(4):

Schmp. 145-147 ° C.

Beispiel X

2-Methyl-2-benzoylaminomethylchromanol-(4), öliges Isomerengemisch.

Beispiel 1

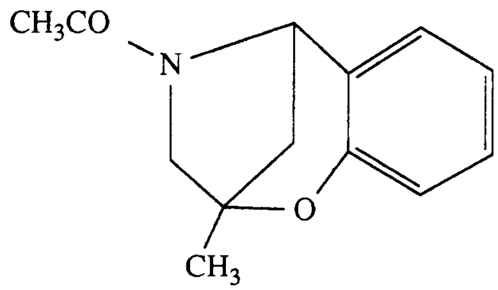

Ein Gemisch von 7,7 g des in Beispiel III erhaltenen Chromanols, 80 ml Chlorbenzol, 80 ml Cyclohexan und 0,5 g p-Toluolsulfonsäure wird am Wasserabscheider zum Sieden erhitzt, wobei eine Klare Lösung entsteht. Nach 20 Minuten gießt man die Lösung auf eine eiskalte Natriumhydrogencarbonat-Lösung, wäscht die organische Phase mit Wasser, trocknet über $Na_2SO_4$,engt ein und erhält 4,3 g 2-Methyl-4-acetyl-2,5-methano-2,3,4,5-tetrahydro-1,4-benzoxazepin. Schmp. 110-112 ° C.
$^1$H-NMR($CDCl_3$): 1,6 (s,$CH_3$ an C-2); 1,81 und 2,05 (2s,$CH_3CO$); 1,9-2,4 (2 überlagerte ABX-Spektren, Methanobrücke); 3,4-3,8 (2 AB-Quartetts, N-$CH_2$); 5,0-5, ppm (2d, H an C-5); 6,7-7,3 (m, 4 aromat. H).

Beispiel 2

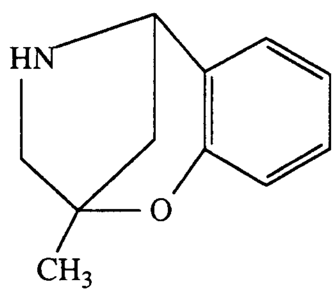

5 g des in Beispiel 1 erhaltenen Benzoxazepins werden in einem Gemisch aus 12 ml 4n NaOH und 10 ml Glyme 10 Stunden bei Rückflußtemperatur verrührt. Nach dem Abkühlen extrahiert man das gebildete Endprodukt mit Chlorbenzol. Nach dem Einengen wird destilliert. Man erhält 2,7 g (67 %) 2-Methyl-2,5-methano-2,3,4,5-tetrahydro-1,4-benzoxazepin (Schmp. 91-3 ° C).
$^1$H-NMR ($CDCl_3$ + $CD_3OD$); 1,62 (s,$CH_3$); 2,1 ($CH_2$,ABX,$\delta_{AB}$ = 11,4 Hz, $\delta_{AX}$ = nicht aufgelöst, $\delta_{BX}$ = 3,5 Hz); 3,2 ($CH_2$-NH,q,$\delta_{AB}$ = 12,0 Hz), 4,12 (CH,d,$\delta_{BX}$ = 3,5 Hz); 6,7-7,2 ppm (4 aromat. H).

Beispiel 3

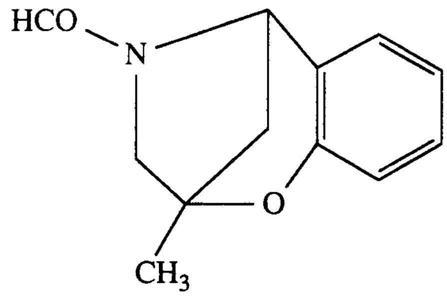

3,5 g des in Beispiel 2 erhaltenen Amins werden in 8 ml Methylenchlorid gelöst und mit 2 ml des gemischten Anhydrids aus Ameisensäure und Essigsäure versetzt. Nach 1 Tag engt man ein, löst den Rückstand in wenig Ether und versetzt mit Petrolether bis zur Trübung. Nach eintägigem Stehen wird abgesaugt. Man erhält 1,6 g 2-Methyl-4-formyl-2,5-methano-2,3,4,5-tetrahydro-1,4-benzoxazepin (Schmp. 103-5 ° C).

Beispiel 4

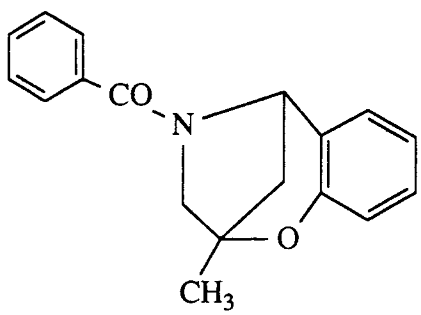

8,5 g des in Beispiel 2 erhaltenen Amins werden in 30 ml THF und 7,3 ml Triethylamin gelöst. Man tropft eine Lösung von 6,8 g Benzoylchlorid in THF zu (durch Eiskühlung hält man die Temperatur unter 30 ° C). Nach 5 Stunden gießt man auf 300 ml Eiswasser, saugt ab und wäscht mit Ether nach.
Ausbeute: 9,1 g (68 %) 2-Methyl-4-benzoyl-2,5-methano-2,3,4,5-tetrahydro-1,4-benzoxazepin (Schmp. 48-50 ° C).

Das gleiche Produkt wird erhalten, wenn man das gemäß Beispiel X gewonnene Chromanol mit p-Toluolsulfonsäure in einem Gemisch aus Chlorbenzol und Cyclohexan (vgl. Beispiel 1) erwärmt.

Beispiel 5

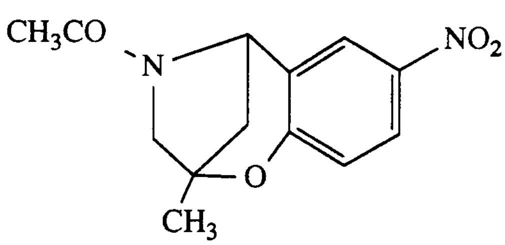

Man löst 21,6 g des in Beispiel 1 erhaltenen Produkts in 100 ml Essigsäureanhydrid, erwärmt auf 50 ° C und gibt innerhalb von 90 min 30 g Kupfer(II)-nitrat-trihydrat in kleinen Portionen bei 50 bis 55 ° C hinzu. Bei der gleichen Temperatur wird noch 2 Stunden nachgerührt und dann der Ansatz auf ein Gemisch von Eiswasser und Xylol gegeben. Man extrahiert zweimal mit Xylol, engt ein und löst den Rückstand in wenig

Ether, worauf das entstandene 2-Methyl-4-acetyl-2,5-methano-2,3,4,5-tetrahydro-7-nitro-benzoxazepin ausfällt Ausbeute: 17,1 g (66 %); Schmp. 122-124 ° C.
$^1$H-NMR ($CDCl_3$): 1,60 (s,$CH_3$); 1,95 und 2,20 (s,$CH_3$-CO), 2,1-2,5 ($CH_2$, 2ABX-Multipletts); 3,6-3,9 (N-$CH_2$, 2AB-Multipletts); 4,99 und 5,32 (d,CH, $\delta$ = 5,5 Hz); 6,8-8,2 (3 aromat. H).

Beispiel 6

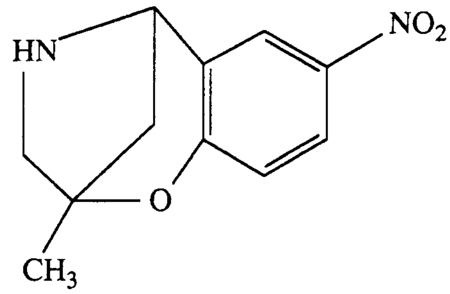

9,8 g der in Beispiel 5 erhaltenen Verbindung werden in 100 ml 2n HCl und 30 ml Glyme 10 Stunden unter Rückfluß erhitzt. Nach dem Abkühlen erhält man 3,8 g (41 %) 2-Methyl-2,5-methano-2,3,4,5-tetrahydro-7-nitro-benzoxazepin als Hydrochlorid (Schmp. 273-276 ° C, Zers.). Die Mutterlauge wird mit 2n NaOH auf einen pH-Wert von 12 eingestellt. Dieses Gemisch extrahiert man mit Ether, engt ein und saugt ab; dabei erhält man 2,2 g (28 %) des obigen Amins in Form der freien Base (Schmp. 102-104 ° C).

Beispiel 7

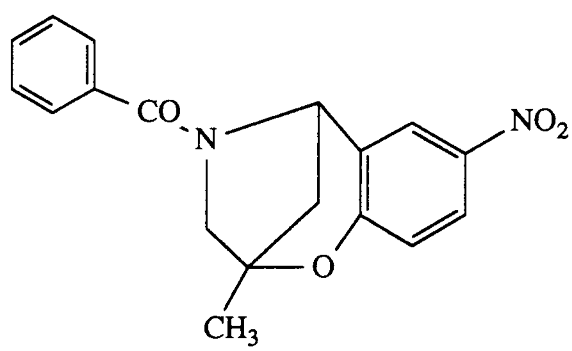

Acylierung des in Beispiel 6 erhaltenen Amins mit Benzoylchlorid analog Beispiel 4 liefert 2-Methyl-4-benzoyl-2,5-methano-2,3,4,5-tetrahydro-7-nitro-benzoxazepin (Schmp. 138-140 ° C).

Beispiel 8

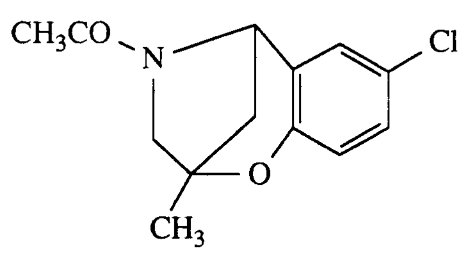

2-Methyl-4-acetyl-2,5-methano-2,3,4,5-tetrahydro-7-chlorbenzoxazepin:

Schmp. 98-100 ° C.

Beispiel 9

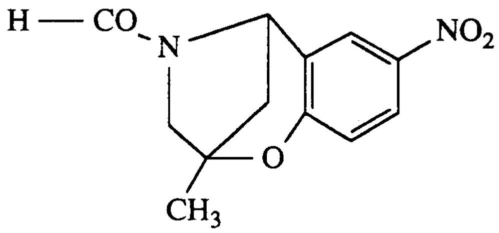

2-Methyl-4-formyl-2,5-methano-2,3,4,5-tetrahydro-7-nitrobenzoxazepin: Schmp. 100-102 ° C.

In analoger Weise werden die folgenden Benzoxazepine erhalten:

Tabelle 1

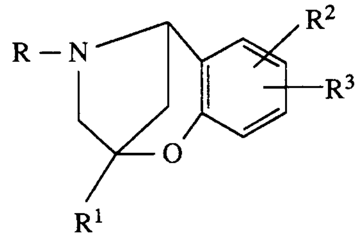

| Beispiel Nr. | R | $R^1$ | $R^2$ | $R^3$ | Ausbeute % d. Th. | Fp. |
|---|---|---|---|---|---|---|
| 10 | $CH_3CO$- | $CH_3$ | 6-Cl | 9-$CH_3$ | 56 | 110°C |
| 11 | 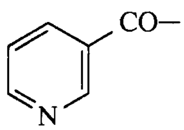 | $CH_3$ | 7-$NO_2$ | H | 67 | 160-162°C |
| 12 | 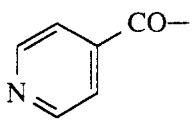 | $CH_3$ | 7-$NO_2$ | H | 58 | 212-214°C |
| 13 | $CH_3$-NH-CO- | $CH_3$ | 7-$NO_2$ | H | 93 | 189-191°C |
| 14 | $CH_3$-NH-CO- | $CH_3$ | H | H | 69 | 168-170°C |
| 15 | 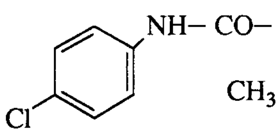 | $CH_3$ | H | H | 81 | 150-152°C |
| 16 | $CH_3$-CO- | $C_2H_5$ | H | H | 29 | Öl |
| 17 | $CH_3$-CO- | 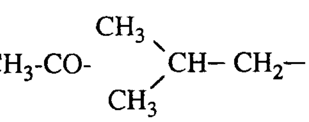 | H | H | 33 | Öl |
| 18 | 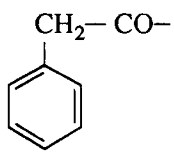 | $CH_3$ | 7-$NO_2$ | H | 65 | 104-107°C |

Tabelle 1 (Fortsetzung)

| Beispiel Nr. | R | $R^1$ | $R^2$ | $R^3$ | Ausbeute % d. Th. | Fp. |
|---|---|---|---|---|---|---|
| 19 | $CH_3$-CO- | $CH_3$ | 7-$CF_3O$ | H | 17 | 107-109°C |
| 20 | -CO-$C(CH_3)_3$ | $CH_3$ | 7-$NO_2$ | H | 86 | 148-150°C |
| 21 | -CO-$CH_2$-CH-$(CH_3)_2$ | $CH_3$ | 7-$NO_2$ | H | 81 | 112-114°C |
| 22 | -CO-$CH_3$ | $CH_3$ | 7-$CF_3O$ | H | 41 | 107-9°C |
| 23 | 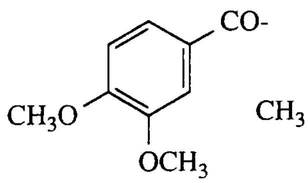 | $CH_3$ | | H | 77 | 140-142°C |
| 24 | 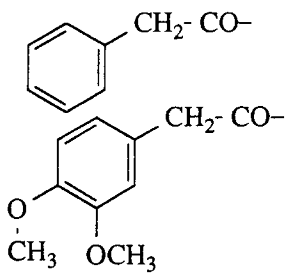 | $CH_3$ | H | H | 74 | 86-88°C |
| 25 | | $CH_3$ | H | H | 49 | Öl |
| 26 | " | $CH_3$ | 7-$NO_2$ | H | 64 | 170-172°C |
| 27 | $CH_3$-CO | $CH_3$ | 6,8(di)$CH_3$ | 7-Cl | 58 | 205-207°C |
| 28 | $CH_3$-CO | $CH_3$ 0 | 6,7-Benzo- | | 61 | 118-120°C |
| 29 | $CH_3$-CO | $CH_3$ | 7-CN | H | 66 | 110-112°C |
| 30 | $CH_2$=CH-$CH_2$-NH-CS- | $CH_3$ | H | H | 90 | 117-119°C |
| 31 | $CH_3$-CO- | $CH_3$ | 8-$C_6H_5$ | H | 48 | 134-137°C |
| 32 | 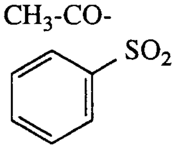 | $CH_3$ | 7-$NO_2$ | H | 82 | 162-164°C |

T a b e l l e 1 (Fortsetzung)

| Beispiel Nr. | R | $R^1$ | $R^2$ | $R^3$ | Ausbeute % d. Th. | Fp. |
|---|---|---|---|---|---|---|
| 33 | $CH_3$-$SO_2$ | $CH_3$ | H | H | 81 | 130-132°C |
| 34 | $CH_3$-CO- | $CH_3$ | 7-F | H | 29 | 58-60°C |
| 35 | $CH_3$-CO | $CH_3$ | 7-F | H | 41 | 97-98°C |
| 36 | $CH_3$-CO | $CH_3$ | 8-$CH_3O$ | H | 89 | 132-134°C |
| 37 | $CH_3$-CO | $CH_3$ | 7-$CH_3SO_2$ | H | 37 | >200°C (Zers.) |
| 38 | H | $CH_3$ | 7-$CF_3O$ | H | 68 | 88-90°C |
| 39 | $CH_3$-CO | $CH_3$ | 7-$CF_3$ | H | 70 | >150°C (Zers.) |
| 40 | $C_2H_5$-CO | $CH_3$ | 7-$NO_2$ | H | 76 | 107-109°C |
| 41 | 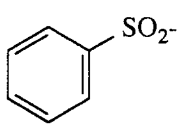 | $CH_3$ | 7-$NO_2$ | H | 85 | 162-164°C |
| 42 | 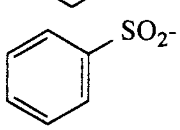 | $CH_3$ | H | H | 91 | 108-110°C |
| 43 | 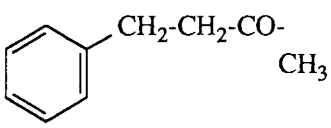 | $CH_3$ | H | H | >90 | Öl |
| 44 | 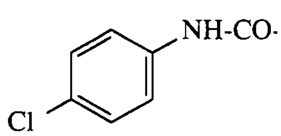 | $CH_3$ | 7-$NO_2$ | H | 93 | 312°C |

Tabelle 1 (Fortsetzung)

| Beispiel Nr. | R | $R^1$ | $R^2$ | $R^3$ | Ausbeute % d. Th. | Fp. |
|---|---|---|---|---|---|---|
| 45 | 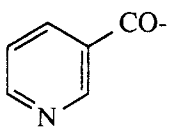 | $CH_3$ | H | H | 42 | 115-117°C |
| 46 | $C_2H_5$-CO- | $CH_3$ | 7-$NO_2$ | H | 54 | 106-109°C |
| 47 | $C_2H_5$O-CO- | $CH_3$ | H | H | 77 | 122-5°C |

## Patentansprüche

**1.** 2,5-Methano-benzoxazepine und -benzthiazepine der allgemeinen Formel (I)

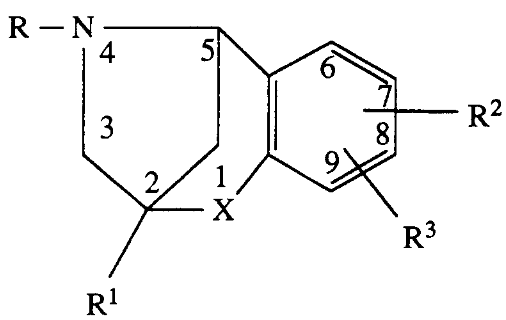

,

in welcher

X für Sauerstoff oder Schwefel steht,

R für Wasserstoff oder eine der Gruppen $COR^4$, $CO\text{-}NHR^4$, $CS\text{-}NHR^4$, $CO\text{-}OR^4$ oder $SO_2R^4$ steht,

wobei

$R^4$ jeweils für Wasserstoff, geradkettiges, verzweigtes oder cyclisches Alkyl mit bis zu 7 C-Atomen, für Alkenyl mit 2 bis 4 C-Atomen, für Phenalkyl mit 7 bis 10 C-Atomen, für Phenyl oder für einen 5- oder 6-gliedrigen Heteroarylrest steht, der ein oder zwei gleiche oder verschiedene Heteroatome aus der Gruppe Sauerstoff, Schwefel oder Stickstoff enthält und wobei die genannten Alkylreste gegebenenfalls durch Halogen substituiert sind und die Phenylreste gegebenenfalls ein-oder zweifach durch Alkyl oder Alkoxy mit jeweils 1 bis 4 C-Atomen oder durch Halogen oder Nitro substituiert sind,

$R^1$ für Wasserstoff oder Alkyl mit 1 bis 4 C-Atomen steht,

$R^2$ and $R^3$ gleich oder verschieden sind und jeweils für ein oder zwei Substituenten aus der Gruppe geradkettiges, verzweigtes oder cyclisches Alkyl mit 1 bis 7 C-Atomen, das gegebenenfalls durch Halogen substituiert ist, Alkoxy mit 1 bis 6 C-Atomen, Halogen, Nitro, Carboxy, Hydroxy, Carbonamid, Alkoxycarbonyl mit bis zu 7 C-Atomen, Alkylsulfonyl mit 1 bis 6 C-Atomen, Phenylsulfonyl oder einen 5- oder 6-gliedrigen Heteroarylrest, der ein oder zwei Heteroatome aus der Gruppe Stickstoff, Sauerstoff

oder Schwefel enthält, für Phenyl, welches gegebenenfalls ein- oder zweifach substituiert ist durch Alkyl oder Alkoxy mit 1 bis 4 C-Atomen, Halogen oder Nitro, oder für $COR^4$ stehen,

oder

$R^2$ gemeinsam mit $R^3$ einen 3- oder 4-gliedrigen anellierten Ring bildet,

sowohl in Form von Isomerengemischen als auch in isomerenreiner Form.

**2.** Verbindungen der allgemeinen Formel (I) gemäß Anspruch 1, in welcher

X für Sauerstoff oder Schwefel steht,

R für Wasserstoff, $COR^4$, $CONHR^4$, $CSNHR^4$, $CO\text{-}OR^4$ oder $SO_2R^4$ steht,

wobei

$R^4$ für Alkyl oder Alkoxy mit bis zu 4 C-Atomen steht, wobei Alkyl gegebenenfalls durch Fluor oder Chlor substituiert, oder für Phenyl steht, das gegebenenfalls durch Alkyl oder Alkoxy mit 1 bis 4 C-Atomen, Fluor, Chlor oder Nitro ein- oder zweifach substituiert ist, oder für Heteroaryl aus der Gruppe Thiophen, Pyridin oder Furan, die gegebenenfalls substituiert sind durch Fluor, Chlor, Methyl, Ethyl oder Methoxy,

$R^1$ für Wasserstoff oder Alkyl mit 1 bis 4 C-Atomen steht,

$R^2$ und $R^3$ gleich oder verschieden sind und jeweils für ein oder zwei Substituenten aus der Gruppe Wasserstoff, Halogen, Nitro, Carboxy, Cyano, Hydroxy, Carbonamid, Phenyl, Phenylsulfonyl, Alkyl mit 1 bis 4 C-Atomen, Alkoxy mit 1 bis 4 C-Atomen, Alkoxycarbonyl mit bis zu 5 C-Atomen, Alkylsulfonyl mit 1 bis 4 C-Atomen, Thiophen, Pyridin oder Furan stehen, wobei die genannten Alkyl-, Aryl-und Heteroarylreste gegebenenfalls substituiert sind durch Fluor, Chlor, Nitro, Alkyl mit 1 bis 4 C-Atomen oder Alkoxy mit 1 bis 4 C-Atomen,

oder wobei $R^2$ und $R^3$ gemeinsam einen 3- oder 4-gliedrigen anellierten Ring bilden.

**3.** Verbindungen der allgemeinen Formel (I) gemäß Anspruch 1,

in welcher

X für Sauerstoff steht,

R für Wasserstoff, $CO\text{-}R^4$, $CO\text{-}NH\text{-}R^4$, $CO\text{-}OR^4$ oder $SO_2\text{-}R^4$ steht, wobei

$R^4$ für Alkyl oder Alkoxy mit 1 bis 4 C-Atomen steht, wobei Alkyl durch Fluor substituiert sein kann,

$R^1$ für Alkyl mit 1 bis 4 C-Atomen steht,

$R^2$ und $R^3$ gleich oder verschieden sind und jeweils für 1 bis 2 Substituenten aus der Gruppe Wasserstoff, Halogen, Nitro, Cyano, Alkyl mit bis zu 4 C-Atomen, Alkylsulfonyl mit bis zu 4 C-Atomen stehen, wobei die genannten Alkylreste durch Fluor substituiert sein können.

**4.** Verfahren zur Herstellung von 2,5-Methano-benzoxazepinen und -benzthiazepinen der allgemeinen Formel (I)

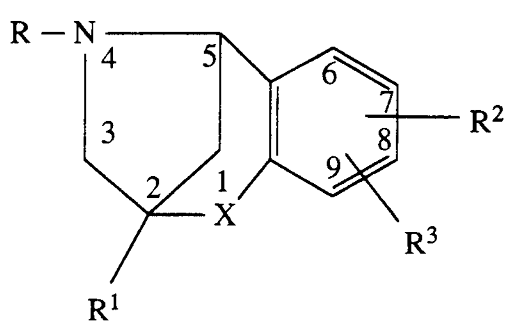

,

in welcher

X für Sauerstoff oder Schwefel steht,

R für Wasserstoff oder eine der Gruppen $COR^4$, $CO\text{-}NHR^4$, $CS\text{-}NHR^4$, $CO\text{-}OR$, $CO\text{-}OR^4$

oder $SO_2R^4$ steht,
wobei

$R^4$ jeweils für Wasserstoff, geradkettiges, verzweigtes oder cyclisches Alkyl mit bis zu 7 C-Atomen, für Alkenyl mit 2 bis 4 C-Atomen, für Phenalkyl mit 7 bis 10 C-Atomen, für Phenyl oder für einen 5- oder 6-gliedrigen Heteroarylrest steht, der ein oder zwei gleiche oder verschiedene Heteroatome aus der Gruppe Sauerstoff, Schwefel oder Stickstoff enthält und wobei die genannten Alkylreste gegebenenfalls durch Halogen substituiert sind und die Phenylreste gegebenenfalls ein-oder zweifach durch Alkyl oder Alkoxy mit jeweils 1 bis 4 C-Atomen oder durch Halogen oder Nitro substituiert sind,

$R^1$ für Wasserstoff oder Alkyl mit 1 bis 4 C-Atomen steht,

$R^2$ und $R^3$ gleich oder verschieden sind und jeweils für ein oder zwei Substituenten aus der Gruppe geradkettiges, verzweigtes oder cyclisches Alkyl mit 1 bis 7 C-Atomen, das gegebenenfalls durch Halogen substituiert ist, Alkoxy mit 1 bis 6 C-Atomen, Halogen, Nitro, Carboxy, Hydroxy, Carbonamid, Alkoxycarbonyl mit bis zu 7 C-Atomen, Alkylsulfonyl mit 1 bis 6 C-Atomen, Phenylsulfonyl oder einen 5- oder 6-gliedrigen Heteroarylrest, der ein oder zwei Heteroatome aus der Gruppe Stickstoff, Sauerstoff oder Schwefel enthält, für Phenyl, welches gegebenenfalls ein- oder zweifach substituiert ist durch Alkyl oder Alkoxy mit 1 bis 4 C-Atomen, Halogen oder Nitro, oder für $COR^4$ stehen,

oder

$R^2$ gemeinsam mit $R^3$ einen 3- oder 4-gliedrigen anellierten Ring bildet,

sowohl in Form von Isomerengemischen als auch in isomerenreiner Form, dadurch gekennzeichnet, daß man Verbindungen der allgemeinen Formel (II)

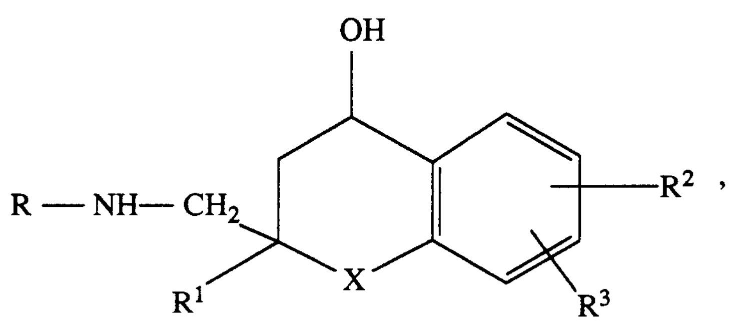

in welcher

X, R, $R^1$, $R^2$ und $R^3$ die oben angegebene Bedeutung haben,

bei Temperaturen zwischen 60 und 200 °C in inerten organischen Lösungsmitteln in Gegenwart von hochsiedenden, wasserfreien Säuren kondensiert und gegebenenfalls das entstehende Wasser azeotrop abdestilliert.

**5.** Verbindungen der allgemeinen Formel (I) gemäß Anspruch 1 zur Verwendung bei der Bekämpfung von Erkrankungen.

**6.** Arzneimittel, enthaltend mindestens eine Verbindung der allgemeinen Formel (I) gemäß Anspruch 1.

**7.** Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

**8.** Verwendung von Verbindungen der allgemeinen Formel (I) gemäß Anspruch 1 bei der Herstellung von kreislaufwirksamen Arzneimitteln.

**9.** Verbindungen der allgemeinen Formel (II)

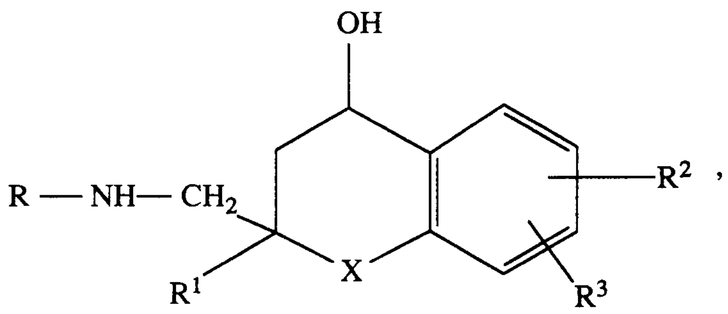

in welcher

X für Sauerstoff oder Schwefel steht,

R für Wasserstoff oder eine der Gruppen $COR^4$, $CO\text{-}NHR^4$, $CS\text{-}NHR^4$, $CO\text{-}OR^4$ oder $SO_2R^4$ steht,

wobei

$R^4$ jeweils für Wasserstoff, geradkettiges, verzweigtes oder cyclisches Alkyl mit bis zu 7 C-Atomen, für Alkenyl mit 2 bis 4 C-Atomen, für Phenalkyl mit 7 bis 10 C-Atomen, für Phenyl oder für einen 5- oder 6-gliedrigen Heteroarylrest steht, der ein oder zwei gleiche oder verschiedene Heteroatome aus der Gruppe Sauerstoff, Schwefel oder Stickstoff enthält und wobei die genannten Alkylreste gegebenenfalls durch Halogen substituiert sind und die Phenylreste gegebenenfalls ein-oder zweifach durch Alkyl oder Alkoxy mit jeweils 1 bis 4 C-Atomen oder durch Halogen oder Nitro substituiert sind,

$R^1$ für Wasserstoff oder Alkyl mit 1 bis 4 C-Atomen steht,

$R^2$ and $R^3$ gleich oder verschieden sind und jeweils für ein oder zwei Substituenten aus der Gruppe geradkettiges, verzweigtes oder cyclisches Alkyl mit 1 bis 7 C-Atomen, das gegebenenfalls durch Halogen substituiert ist, Alkoxy mit 1 bis 6 C-Atomen, Halogen, Nitro, Carboxy, Hydroxy, Carbonamid, Alkoxycarbonyl mit bis zu 7 C-Atomen, Alkylsulfonyl mit 1 bis 6 C-Atomen, Phenylsulfonyl oder einen 5- oder 6-gliedrigen Heteroarylrest, der ein oder zwei Heteroatome aus der Gruppe Stickstoff, Sauerstoff oder Schwefel enthält, für Phenyl, welches gegebenenfalls ein- oder zweifach substituiert ist durch Alkyl oder Alkoxy mit 1 bis 4 C-Atomen, Halogen oder Nitro, oder für $COR^4$ stehen,

oder

$R^2$ gemeinsam mit $R^3$ einen 3- oder 4-gliedrigen anellierten Ring bildet.

Europäisches Patentamt

# EUROPÄISCHER RECHERCHENBERICHT

Nummer der Anmeldung

EP 92 10 0415

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | GB-A-2 194 786 (DR. MADAUS GMBH & CO)<br>* Seite 13, Zeile 35 - Seite 13, Zeile 46; Ansprüche 1,11 *<br>--- | 1,6,8 | C07D491/08<br>C07D495/08<br>C07D311/22<br>C07D335/06<br>A61K31/55<br>//(C07D491/08, 311:00,209:00)<br>(C07D495/08, 335:00,209:00) |
| P,X | ANGEWANDTE CHEMIE. INTERNATIONAL EDITION.<br>Bd. 30, Nr. 12, Dezember 1991, WEINHEIM DE<br>Seiten 1709 - 1711;<br>H. J. KABBE ET AL.: '4-Acyl-2,3,4,5-tetrahydro-2-methyl-2,5-methanobenz-1,4-oxazepine, derivatives of a new heterocycle'<br>* das ganze Dokument *<br>--- | 1 | |
| P,X | EP-A-0 415 065 (MERCK)<br>* Seite 12, Zeile 25 - Seite 12, Zeile 30 *<br>----- | 9 | |

| RECHERCHIERTE SACHGEBIETE (Int. Cl.5 ) |
|---|
| C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 29 APRIL 1992 | VOYIAZOGLOU D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument